# EUROPÄISCHE PATENTSCHRIFT

(11) **EP 1 696 904 B1**
(45) Veröffentlichungstag und Bekanntmachung des Hinweises auf die Patenterteilung: **16.11.2011**
(21) Anmeldenummer: 04804249.3
(22) Anmeldetag: 23.12.2004
(51) Int. Cl.: A61K 31/381, A61P 25/16

(54) **VERWENDUNG VON ROTIGOTIN ZUR BEHANDLUNG ODER ZUR PRÄVENTION DES DOPAMINERGEN NEURONENVERLUSTES**
USE OF ROTIGOTINE FOR THE TREATMENT OR PREVENTION OF DOPAMINERGIC NEURONE LOSS
UTILISATION DE ROTIGOTINE POUR TRAITER OU PREVENIR LA PERTE DES NEURONES DOPAMINERGIQUES

(30) Priorität: 24.12.2003 DE 10361259
(43) Veröffentlichungstag der Anmeldung: 06.09.2006
(73) Patentinhaber: UCB Pharma GmbH, 40789 Monheim (DE)
(72) Erfinder: SCHELLER, Dieter, 41470 Neuss (DE); DRESSEN, Frank, 52391 Vettweiss (DE)
(74) Vertreter: HOFFMANN EITLE
(86) Internationale Anmeldenummer: PCT/EP2004/014655
(87) Internationale Veröffentlichungsnummer: WO 2005/063237

(56) Entgegenhaltungen:
- EP-A1- 1 386 605
- WO-A-02/31499
- WO-A-03/012137
- WO-A1-2005/092331
- TUITE P ET AL: "RECENT DEVELOPMENTS IN THE PHARMACOLOGICAL TREATMENT OF PARKINSON'S DISEASE" EXPERT OPINION ON INVESTIGATIONAL DRUGS, ASHLEY PUBLICATIONS LTD., LONDON, GB, Bd. 12, Nr. 8, 1. August 2003 (2003-08-01), Seiten 1335-1352, XP009023949 ISSN: 1354-3784
- GERLACH M ET AL: "Arguments for the use of dopamine receptor agonists in clinical and preclinical Parkinson's disease" JOURNAL OF NEURAL TRANSMISSION, SUPPLEMENT 2003 AUSTRIA, Nr. 65, 2003, Seiten 167-183, XP009046204 ISSN: 0303-6995
- IZAAK DEN DAAS ET AL.: "Improvement of the oral bioavailability of the selective dopamine agonist N-0437 in rats: the in vitro and in vivo activity of eight ester prodrugs" NAUNYN-SCHMIEDEBERG S ARCH PHARMACOL, Bd. 341, 1990, Seiten 186-191, XP009046188
- TUITE P ET AL: "RECENT DEVELOPMENTS IN THE PHARMACOLOGICAL TREATMENT OF PARKINSON'S DISEASE", EXPERT OPINION ON INVESTIGATIONAL DRUGS, ASHLEY PUBLICATIONS LTD., LONDON, GB, vol. 12, no. 8, 1 August 2003 (2003-08-01) , pages 1335-1352, XP009023949, ISSN: 1354-3784, DOI: DOI:10.1517/EOID.12.8.1335.21771
- GERLACH M ET AL: "ARGUMENTS FOR THE USE OF DOPAMINE RECEPTOR AGONISTS IN CLINICAL AND PRECLINICAL PARKINSON'S DISEASE", JOURNAL OF NEURAL TRANSMISSION. SUPPLEMENTUM, VIENNA, AT, no. 65, SUPPL, 1 January 2003 (2003-01-01) , pages 167-183, XP009046204, ISSN: 0303-6995
- DAAS DEN I ET AL: "IMPROVEMENT OF THE ORAL BIOAVAILABILITY OF THE SELECTIVE DOPAMINE AGONIST N-0437 IN RATS: THE IN VITRO AND IN VIVO ACTIVITY OF EIGHT ESTER PRODRUGS", NAUNYN-SCHMIEDEBERG'S ARCHIVES OF PHARMACOLOGY, SPRINGER, BERLIN, DE, vol. 341, 1 January 1990 (1990-01-01), pages 186-191, XP009046188, ISSN: 0028-1298, DOI: DOI:10.1007/BF00169729
- BECKER, G. ET AL.: J. NEUROL., vol. 249 (Suppl.3), 1 January 2002 (2002-01-01), pages III/40-III/48,

## Beschreibung

Die Parkinson'sche Krankheit ist die Folge einer chronisch-fortschreitenden Degeneration von Neuronen, deren Ursache noch nicht völlig aufgeklärt ist. Sie wird klinisch manifest in Form der Kardinalsymptome Ruhetremor, Rigor, Bradykinesie und posturale Instabilität.

Als Medikamente zur Linderung der motorischen Symptome werden in erster Linie Levodopa, Dopaminagonisten, wie z.B. Rotigotin, Pramipexol, Bromocriptin, Ropinirol, Cabergoline, Pergolid, Apomorphin und Lisurid, Anticholinergika, NMDA-Antagonisten, β-Blocker sowie der MAO-B-Inhibitor Selegelin und der COMT-Inhibitor Entacapone eingesetzt. Diese meisten dieser Wirkstoffe greifen in die dopaminerge und/oder cholinerge Signalkaskade ein und beeinflussen auf diese Weise symptomatisch die Parkinson-typischen Bewegungsstörungen.

Die bisherige Therapie der Parkinson'schen Krankheit setzt beim Eintreten der Kardinalsymptome ein. Eine Morbus Parkinson-Erkrankung gilt klinisch im allgemeinen als nachgewiesen, wenn mindestens zwei der vier Kardinalsymptome (Bradykinesie, Ruhetremor, Rigor und posturale Instabilität) zu finden sind und L-Dopa anspricht (Hughes, J Neurol Neurosurg Psychiatry 55,1992,181). Unglücklicherweise treten die motorischen Störungen bei Morbus Parkinson-Patienten aber erst auf, wenn ca. 70-80 % der dopaminergen Neurone in der Substanzia Nigra (SN) irreversibel geschädigt sind (Becker et al, J Neurol 249, 2002, Suppl 3:III, 40; Hornykiewicz, Encyclopedia of Life Science 2001, 1). Die Chance einer nachhaltigen Therapie zu diesem Zeitpunkt sind nur noch gering. Daher ist es wünschenswert, die Therapie so früh wie möglich einsetzen zu lassen.

Aktuelle klinische Beobachtungen sowie anatomische und genetische Untersuchungen zeigen nun, dass sowohl die Diagnose von Morbus Parkinson-Patienten in frühen Stadien als auch die Identifizierung von Risikopatienten möglich ist.

Als diagnostische Marker können dabei beispielsweise herangezogen werden:
- Biochemische Marker, wie Neuromelanin (Gerlach, Neurotox Res 5,2003, 35; WO 02/31499), S-100 beta (Muramatsu, Glia 42, 2003, 307), das alpha-Synuclein (WO 03/069332; WO 00/02053) oder das parkin-Protein (Sharma, Neurol Clin N Am 20, 2002, 759) sowie Semaphorin (Wo 03/007803)
- Genetische Marker, z.B. die Park-Gene 1-8 (Guttman, CMAJ 4, 2003, 168; WO 03/76658); CYP2D6-B (WO 03/012137), Chromosom 2q 36-37 (Pankratz, Am J Hum Gen 72, 2003,e-pub), a-Synuclein (Polymeropoulos, Science. 276, 1997, 2045) oder Mutationen in CYP2D6-B und GSTM1-Deletion (WO 03/012137).
- Bildgebende Verfahren, wie die Ultraschalluntersuchung der SN-Größe, gegebenenfalls in Kombination mit anderen Verfahren (Becker et al, J Neurol 249, 2002, Suppl 3:III, 40) oder MRI (Hutchinson M, Raff U., J Neurol Neurosurg Psychiatry. 1999 Dec; 67(6):815-8).
- Bildgebende Verfahren wie PET oder SPECT (Prunier C. Bezard E, et al., Neuroimage. 2003 Jul;19(3):810-6). - Sensorische Störungen oder Verhaltensauffälligkeiten, wie Schlaf- und Riechstörungen, insbesondere Schlafstörungen vom Typ der ,REM Sleep Behavior Disorder', (Henderson, J Neurol Neurosurg Psychiatry 74, 2003, 956) oder kognitive Abnormalitäten (Rammsayer, Int J Neurosci. 91,1997,45).
- Organische Probleme, wie Obstipation (Krygowska-Wajs, Funct Neurol 15,2000,41).
- Depression (Camicioli R. Drugs Today (Barc). 2002 Oct; 38(10):677-86).
- Kurzfristige Bewegungsanomalien, wie z.B. Chorea oder orthostatische Auffälligkeiten
- Kombinationen aus oben genannten Markern (Stern, Annals of Neurol 56, 2004, 169)

Dadurch eröffnet sich die Chance, den Krankheitsprozess zu einem Zeitpunkt zu beeinflussen, zu dem noch mehr Neurone vorhanden sind, als zum Zeitpunkt des Auftretens mehrerer motorischer Kardinalsymptome der Parkinson'schen Krankheit und damit eine quantitativ höhere Anzahl von Neuronen zu schützen. Eine Gabe eines effektiven Neuroprotektivums zu einem frühen Stadium lässt erwarten, dass der Krankheitsprozess deutlich verzögert werden kann: Je früher eine Therapie einsetzen kann, umso höher sind die Chancen eine langanhaltende Verhinderung des Beginns der die Lebensqualität einschränkenden Symptome zu erreichen.

Es besteht daher der Bedarf an Arzneimitteln, die nicht nur die dopaminerge Übertragung beeinflussen und die Symptomatik der Parkinson'schen Krankheit in fortgeschrittenen Stadien lindern können, sondern die den fortschreitenden Untergang dopaminerger Neuronen in frühen, motorisch weitgehend asymptomatischen Parkinsonstadien umkehren, verhindern oder zumindestens deutlich verlangsamen können (Dawson, Nature Neuroscience Supplement 5, 2002, 1058).

Rotigotin [(-)-5,6,7,8-Tetrahydro-6-[propyl[2-(2-thienyl)ethyl]amino]-1-naphthol] ist aus dem Stand der Technik als Dopaminagonist und rein symptomatisches Morbus Parkinson-Therapeutikum bekannt. Die WO 02/089777 beschreibt beispielsweise die transdermale Gabe von Rotigotin bei Parkinson-Patienten und die damit verbundene Verbesserung des UPDRS (Unified Parkinson's-Disease Rating-Scale)-Scores. Der UPDRS-Score ist ein wichtiges Instrument zur Diagnose und zum Monitoring der Progression bzw. der Therapie von Morbus Parkinson-Patienten (Fahn S, Elton RL, Members of the UPDRS Development Committee (1987) Unified Parkinson's Disease Rating Scale. In: Fahn, S, CD Marsden, DB Calne, M Goldstein (eds) Recent Devlopments in Parkinson's Disease. Vol II. Macmillan Healthcare Information, Florham Park (NJ), pp 153-163, 293-304). Allerdings wird mit dem UPDRS-Score lediglich der Effekt eines Wirkstoffs auf die Parkinson-Symptomatik erfasst. Er erlaubt keine Aussagen darüber, ob ein Wirkstoff den der Symptomatik zugrunde liegenden dopaminergen Zelluntergang beeinflusst.

Metman et al (Clin Neuropharmacol 24,2001, 163) beschreiben ebenfalls die Wirkung von Rotigotin auf Parkinson-assoziierte Bewegungsstörungen. Die behandelten Patienten hatten bereits ausgeprägte Dyskinesien, die durch Rotigotin-Gabe verbessert wurden.

Aus dem Stand der Technik ist Rotigotin somit als Dopamin-Agonist zur symptomatischen Behandlung von Morbus Parkinson bekannt. Rein symptomatisch wirkende Parkinson-Arzneimittel versprechen jedoch bei der vorbeugenden Behandlung von Morbus Parkinson keinen Vorteil, da sie keinen Einfluß auf den dopaminergen Zelluntergang und auf das Fortschreiten bzw. die Ausprägung der Krankheit haben.

Experimentelle Untersuchungen zeigten nun aber überraschend, dass das bisher nur zur symptomatischen Therapie der Parkinson'schen Krankheit eingesetzte Rotigotin neuroprotektive Eigenschaften besitzt, bzw. die regenerative Kapazität neuronaler Elemente fördert, so daß Rotigotin als Arzneimittel zur Verhinderung des dopaminergen Zellverlusts, auch und insbesondere in sehr frühen Stadien der Parkinson'schen Krankheit oder bei Risikokandidaten eingesetzt werden kann.

### Abbildungen

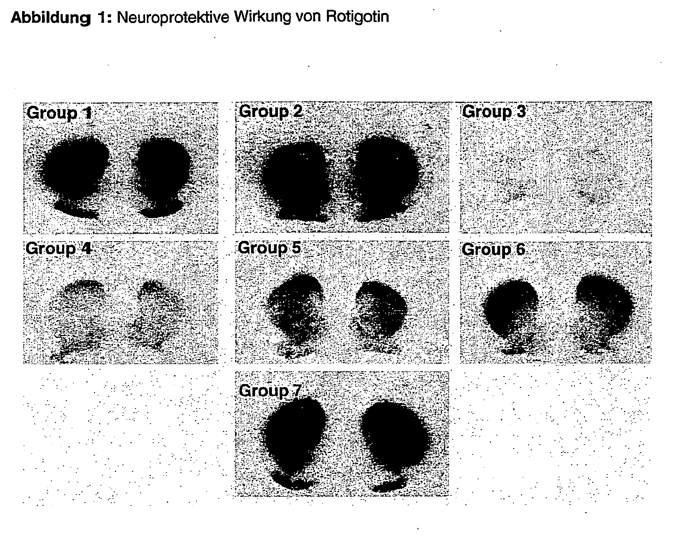

Abbildung 1 zeigt repräsentative Beispiele für die neuroprotektive Wirkung von Rotigotin gemessen an der Dichte der Dopamin-Transporter als Indiz für die Dichte der verbliebenen Nervenendigungen im Striatum

Die Gruppen 1-7 wurden wie folgt behandelt: Gruppe 1: unbehandelte Kontrolle; Gruppe 2: Kontrolle behandelt mit VehikeHösung für Rotigotin und MPTP; Gruppe 3: MPTP-Behandlung; Gruppe 4: MPTP-Behandlung plus Rotigotin 0.3 mg/kg; Gruppe 5: MPTP-Behandlung plus Rotigotin 1.0 mg/kg; Gruppe 6: MPTP-Behandlung plus Rotigotin 3.0 mg/kg; Gruppe 7: Behandlung nur mit Rotigotin (3.0 mg/kg)

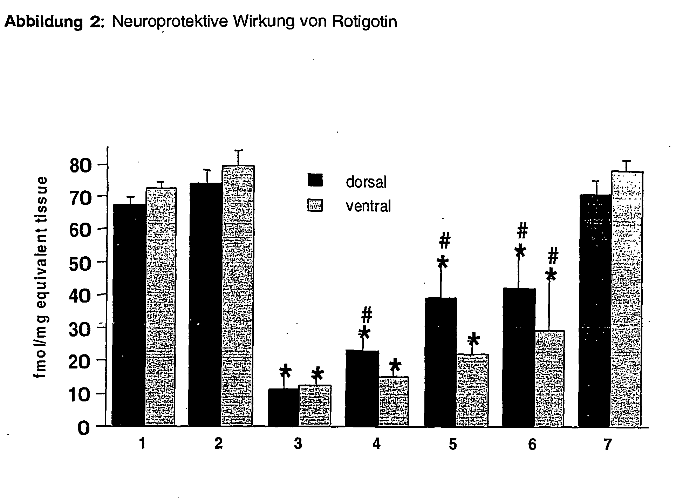

Abbildung 2 zeigt die Dopamin-Transporter (DAT) Bindung im dorsalen und ventralen Teil des Striatums in verschiedenen Gruppen durch Quantifizierung der DAT-Dichte nach einem Experiment wie in Abbildung 1 illustriert. Die Balkendiagramme 1-7 entsprechen den in Abbildung 1 wiedergegebenen Gruppen 1-7. Die mit * gekennzeichneten Gruppen zeigten einen signifikanten Abfall in der DAT Bindung im Vergleich zur Kontrollgruppe 2. Die mit # gekennzeichneten Gruppen wiesen einen signifikanten Erhalt der DAT Bindung im Vergleich mit der MPTP-behandelten Gruppe 3 auf.

### Beschreibung der Erfindung

Apoptotische Vorgänge sollen beim Untergang dopaminerger Neurone in der Parkinson-Pathogenese eine wichtige Rolle spielen (Barzilai, Cell Mol Neurobiol 21, 2001, 215). Es werden daher neuroprotektive Substanzen gewünscht, die den dopaminergen Zelluntergang stoppen oder sogar umkehren können. Als prädiktiv für die geforderten neuroprotektiven Eigenschaften gilt dabei das MPTP-Modell (Dawson, Nature Neuroscience Supplement 5, 2002, 1058).

Rotigotin zeigt sowohl in einem akuten wie in einem subakuten MPTP-Modell überraschend das gewünschte pharmakologische Profil. Die Untersuchungsergebnisse legen nahe, dass mit Rotigotin apoptotische Prozesse verhindert werden.

Rotigotin zeigt beispielsweise neuroprotektive Wirkung in einem Parkinsonmodell der Maus: Nach akuter Gabe von MPTP, das bei Menschen wie bei Affen ein Parkinson-Syndrom erzeugt, wurde zum einen die Anzahl der in der akuten Phase degenerierenden Neurone gemessen (Tabelle 1) und zum anderen die funktionelle Integrität des Striatums in der subaktuen Phase durch Bestimmung der Dichte des Dopamin-Transporters in den terminalen Nervenendigungen erfasst (Abbildungen 1 und 2). In beiden Fällen konnte gezeigt werden, dass Rotigotin neuroprotektiv wirksam war: Zum einen war die Anzahl degenerierender Neurone im Mesenzephalon nach der Gabe von Rotigotin-verringert und zum anderen war die dopaminerge Innervation des Striatums nahezu vollständig erhalten bzw. wieder hergestellt.

**Tabelle 1: Anzahl degenerierender Neurone bei der Maus, dargestellt mit FluoroJade Färbung**

| Gruppe | Anzahl degen. Neurone | Stndrtabw |
|---|---|---|
| 1: Vehikel-behandelte Kontrolle | 2.0 | 2.4 |
| 2: MPTP Intoxikation | 73.5 | 34.0 |
| 3: MPTP Intoxikation + Rotigotin 0.3 mg/kg | 66.7 | 30.5 |
| 4: MPTP Intoxikation + Rotigotin 1.0 mg/kg | 76.8 | 41.6 |
| 5: MPTP Intoxikation + Rotigotin 3.0 mg/kg | 34.9 | 31.9 |
| 5: MPTP -Vehikel + Rotigotin 3.0 mg/kg | 3.8 | 4.3 |

In einer Pilotstudie wurde auch die neuroprotektive Wirkung von Rotigotin an Affen untersucht.

In dem verwendeten Modell, das den progressiven Verlauf der Parkinson'schen Erkrankung an Primaten widerspiegelt, wurden Affen (Makaken) unterschwellig-toxische Dosen von MPTP über mehrere Tage injiziert. Die Parkinson-Symptomatik entwickelte sich in dem Modell über einen Zeitraum von ca 2 Wochen. Sobald ein bestimmter Grad der Schädigung erreicht war, wurden tägliche Injektionen von Rotigotin in einer Formulierung vorgenommen, die einen kontinuierlichen Plasmaspiegel über 24 h erzeugt. Die Injektionen von MPTP wurden gestoppt, sobald die motorische Aktivität um einen bestimmten Grad vermindert war (ca 5 Tage später). Das Verhalten der Tiere wurde täglich bewertet. Sechs Wochen nach Beginn der MPTP-Applikation wurden die Injektionen von Rotigotin gestoppt und die Tiere wurden für weitere zwei Wochen ohne Behandlung beobachtet. Es wurde beobachtet, daß die motorische Aktivität der Tiere unter Behandlung und auch in der folgenden Auswaschphase deutlich verbessert war.

Am Ende der Rotigotin-Applikation bzw. am Ende der Auswaschphase wurde je eine Gruppe von Tieren getötet und der Zustand der Basalganglien histologisch und biochemisch untersucht. Die Dichte der Nervenendigungen im Striatum war gegenüber den unbehandelten Tieren deutlich erhöht. Der Gehalt an Pre-Pro-Enkephalin, ein Indikator für die intakte Vernetzung im 'indirect pathway' der Basalganglien, zeigte nach der Behandlung und nach der Auswaschphase eine Tendenz zur Normalisierung.

Die Resultate zeigen, daß das neuroprotektive Potential von Rotigotin auch in einem Primatenmodel von Parkinson'scher Erkrankung nachgewiesen werden kann. Damit kann eine neuroprotektive Wirkung auch beim Menschen angenommen werden.

Mit Rotigotin wurde damit der Therapie ein Wirkstoff zur Verfügung gestellt, der in idealer Weise zur Herstellung eines Arzneimittels, zur Prävention des dopaminergen Neuronenverlustes bei neurodegenerativen Erkrankungen geeignet ist.

Ein Gegenstand der vorliegenden Anmeldung ist daher Rotigotin zur Verwendung bei der Behandlung des dopaminergen Neuronenverlusts bei Patienten, die an einer neurodegenerativen Erkrankung leiden, die mit einem erhöhten dopaminergen Zelluntergang verbunden ist.

Ein erhöhter dopaminerger Neuronenverlust tritt regelmäßig bei Morbus Parkinson-Patienten auf, wird jedoch auch häufig bei anderen neurodegenerativen Erkrankungen, z.B. bei alpha-Synucleopathien oder der Huntington'schen Erkrankung sowie bei REM-Schlafstörungen und Störungen des Geruchssinns beobachtet.

Im Vergleich zur bisherigen Anwendung von Rotigotin, die auf die rein symptomatische Behandlung von Morbus Parkinson-Patienten mit Bewegungsstörungen beschränkt war, wird als neues Anwendungsgebiet insbesondere die prophylaktische Behandlung von Individuen erschlossen, die sich in einem Frühstadium der Parkinson'schen Erkrankung befinden, bzw. die für Morbus Parkinson auf Grund genetischer oder anderer Risiken prädisponiert sind. Wie bereits weiter oben beschrieben, profitieren solche Individuen besonders von der neuroprotektiven Wirkung von Rotigotin, da durch die Rotigotin-Gabe der dopaminerge Zellverlust zu einem Zeitpunkt gestoppt oder verlangsamt wird, zu dem noch eine höhere Zahl dopaminerger Neuronen vorhanden ist, als dies bei Patienten der Fall ist, die bereits motorische Symptome aufweisen.

Ein Gegenstand der Erfindung ist daher die Verwendung von Rotigotin oder seinen Salzen und Prodrugs als Arzneimittel zur vorbeugenden Behandlung des dopaminergen Zellverlusts von Individuen, bei denen vor Eintritt der vorbeugenden Behandlung wenigstens drei der vier Morbus Parkinson-Kardinalsymptome aus der Gruppe Bradykinesie, Rigor, Ruhetremor und posturale Instabilität noch nicht vorhanden sind.

Die Individuen können dabei auch gesund scheinende Individuen sein, deren genetische oder epidemische Prädisposition kein erhöhtes Risiko einer Parkinson-Erkrankung erkennen lassen.

Für eine Rotigotin-Behandlung in Frage kommen aber insbesondere Individuen mit erhöhtem Risiko für Morbus Parkinson oder aber Patienten, bei denen klinische, klinischchemische oder klinisch-pysikalische Morbus Parkinson-Frühsymptome nachweisbar sind, ohne dass diese Patienten jedoch bereits zwei oder mehrere der Kardinalsymptome der Parkinson'schen Krankheit aufweisen.

Schließlich kann Rotigotin als Neuroprotektivum auch dann angewendet werden, wenn die Diagnose nicht eindeutig ist, aber eine Entwicklung einer Symptomatik in Richtung Parkinson-ähnlicher Neurodegeneration erwarten lässt.

Der Prävention des neuronalen dopaminergen Zellverlusts bedürfen insbesondere
(a) Individuen mit einem erhöhten Risiko für Morbus Parkinson oder
(b) Individuen mit Frühsymptomatik für Morbus Parkinson

Die Begriffe "Morbus Parkinson" und "Parkinson'sche Krankheit" werden in dieser Patentanmeldung als Synonyme verwendet und umfassen idiopathischen und genetischen Parkinson. Davon abzugrenzen ist das sogenannte Parkinson-Plus-Syndrom sowie der sekundäre Parkinsonismus.

Unter dem Begriff "Kardinalsymptome" von Morbus Parkinson werden in dieser Patentanmeldung eines oder mehrere der Symptome Bradykinesie, Rigor, Ruhetremor und posturale Instabilität verstanden.

Unter "Individuen mit einem erhöhten Risiko für Morbus Parkinson" werden in dieser Patentanmeldung insbesondere Individuen verstanden, die noch keine nachweisbaren Symptome der Parkinson'schen Krankheit aufweisen, die aber bestimmte Risikofaktoren aufweisen.

Solche Risikofaktoren können genetische Mutationen sein (Nussbaum NEJM 348,2003, 25). Beispielsweise wird das Parkin-Gen auf Chromosome 6q25.2-27 (PARK2) assoziiert mit juvenilem Parkinsonismus und tritt in Familien mit autosomal rezessiver Parkinson-Vererbung verstärkt auf (Matsumine, Am. J. Hum. Genet., 60, 1997, 588; Kitada, Nature 392, 1998, 605; Abbas, Hum. Mol. Genet. 8, 1999, 567; Tassin, Am. J. Hum. Genet., 63, 1998, 88 und Lucking, N. Engl. J. Med. 342, 2000, 1560-7). Weitere Gen-Loci, z.B. PARK6 and PARK7 wurden ebenfalls vermehrt in Familien mit juveniler, rezessiv vererbter Parkinson'schen Krankheit gefunden (Valente, Am. J. Hum. Genet. 68, 2001,895; van Dujin, Am. J. Hum. Genet. 69, 2001, 629). Das PARK8-Gen wird hingegen mit dem späten Auftreten von Morbus Parkinson assoziiert (WO 03/076658). Mutationen im alpha-synuclein-Gen (PARK1) wurden in Familien mit juvenilem, autosomal dominant vererbtem Morbus Parkinson nachgewiesen (Polymeropoulos, Science 276,1997, 2045).

Unter "Individuen mit Parkinson-Frühsymptomatik" werden in dieser Patentanmeldung insbesondere solche Individuen verstanden, bei denen mindestens drei der vier Kardinalsymptome (Rigor, Ruhetremor, Bradykinesie und posturale Instabilität) noch nicht vorhanden sind, die aber diagnostisch nutzbare klinische, klinisch-biochemische und/oder klinisch-pysikalische Frühsymptome aufweisen. Klinisch-biochemische Marker können Veränderungen des alpha-Synuclein oder Neuromelanin-Musters (WO 02/31499) sein. Solche Veränderungen können z.B. beruhen auf der Expression genetischer Varianten z.B. des alpha Synucleins, der Entstehung von Aggregaten oder Filamenten, z.B. des alpha-Synucleins oder der verstärkten Freisetzung aus zellulären Speichern, z.B. aus dem Cytoplasma untergehender Zellen, wie beim Neuromelanin.

Klinisch-physikalische Frühsymptome können strukturelle oder funktionelle Änderungen des Gehirns sein, die sich beispielsweise physikalisch durch PET- und SPECT-Studien oder durch transkranielle Sonographie (Becker, J Neurol 249, Suppl 3, 2002,111/40; Prunier C, et al., Neuroimage. 2003 Jul; 19(3):810-6) nachweisen lassen.

Klinische Frühsymptome können Riechstörungen, Depressionen, Obstipation, Störungen visueller und kognitiver Funktionen oder bestimmte Formen von Schlafstörungen, insbesondere vom Typ der "REM Sleep Behavior Disorders" sein, wobei zur Frühdiagnose auch eine Mischung verschiedener Tests herangezogen werden kann (Becker, J Neurol 249, Suppl 3, 2002, III/40; Stern, Annals of Neurol 56, 2004, 169).

Wie bereits weiter oben diskutiert, sind zum Zeitpunkt des ersten Auftretens von mindestens zwei der vier Kardinalsymptome bereits ca 70-80% der dopaminergen Neuronen der Substantia nigra untergegangen. Um das überraschende neuroprotektive Potenzial von Rotigotin effektiv zu nutzen, setzt die prophylaktische Behandlung der Patienten daher bevorzugt in einem Stadium ein, in dem die Patienten einen geringeren Verlust dopaminerger Zellen der Substantia Nigra (SN) aufweisen. Erfindungsgemäß werden daher Individuen mit Rotigotin behandelt, die erst eines oder noch keines der Kardinalsymptome der Parkinson'schen Krankheit in deutlich ausgeprägter Form aufweisen.

Bevorzugt werden Individuen behandelt, die einen dopaminergen Zellverlust in der SN von weniger als 70 %, 60%, 50% und besonders bevorzugt von weniger als 40%, 30%, 20% oder 10% aufweisen.

Als Hilfsmittel zur Diagnose und zur Therapiekontrolle bei motorisch bereits auffälligen Patienten können zwei Scores herangezogen werden: Der UPDRS-Score und der Hoehn und Yahr-Score.

In einem bevorzugten Aspekt der Erfindung weist das prophylaktisch mit Rotigotin behandelte Patientenkollektiv einen modifizierten Hoehn und Yahr-Score von 0 bis 2, besonders bevorzugt von 0 bis 1 und ganz besonders bevorzugt von 0 auf.

| |
|---|
| Tabelle 2: Modifizierte Stadienbestimmung nach Hoehn, The natural history of Parkinson's disease in the pre-levodopa and post-levodopa eras. Neurologic Clinics 10, 1992, 331 |
| |
| Stadium 0 = Keine Anzeichen der Erkrankung. |
| Stadium 1 = Einseitige Erkrankung. |
| Stadium 1.5 = Einseitige und axiale Beteiligung. |
| Stadium 2 = Beidseitige Erkrankung ohne Gleichgewichtsstörung. |
| Stadium 2.5 = Leichte beidseitige Erkrankung mit Ausgleich beim Zugtest. |
| Stadium 3 = Leichte bis mäßige beidseitige Erkrankung: |
| leichte Haltungsinstabilität; körperlich unabhängig. |
| Stadium 4 = Starke Behinderung; kann noch ohne Hilfe laufen oder stehen. |
| Stadium 5 = Ohne Hilfe an den Rollstuhl gefesselt oder bettlägerig. |

Üblicherweise werden Patienten mit einem UPDRS-Score, Teil III (siehe Ausführungsbeispiel 5) von mindestens 10 als für die dopaminerge Therapie in Frage kommend eingestuft. Das zur Nutzung der neuroprotektiven Wirkung von Rotigotin geeignete Patientenkollektiv weist aber bevorzugt einen sehr niedrigen oder nicht nachweisbaren motorischen UPDRS-Score (Teil III) auf. Im Sinne der vorliegenden Erfindung sollte die vorbeugende Behandlung mit Rotigotin daher bevorzugt bei Patienten erfolgen, die einen UPDRS Motor-Score von weniger als 10, 9, 8, 7, 6, 5, 4, 3, 2 oder 1 aufweisen. Besonders bevorzugt weisen die Patienten noch keinerlei motorische Störungen auf.

Die Begriffe "Prävention", "Prophylaxe" und "vorbeugende Behandlung" werden in dieser Patentanmeldung als Synonyme verwendet. Sie umfassen die Gabe eines Arzneimittels an Individuen, bei denen ein dopaminerger Zellverlust zu erwarten oder bereits aufgetreten ist. Dies sind insbesondere Patienten, bei denen mindestens drei der vier Kardinalsymptome von Morbus Parkinson (Rigor, Ruhetremor, Bradykinesie, posturale Instabilität) noch nicht vorhanden sind, um das Auftreten oder die signifikante Ausprägung der motorischen Symptomatik der Parkinson'schen Krankheit und/oder weiteren dopaminergen Neuronenverlust, insbesondere in der Substantia nigra, zu verhindern oder zu verzögern. Bevorzugt weisen die prophylaktisch zu behandelnden Individuen noch keines der Kardinalsymptome in deutlich ausgeprägter Form vor. Die prophylaktisch zu behandelnden Individuen weisen bevorzugt einen UPDRS-Score von weniger als 10, weniger als 9, 8, 7 oder 6 und besonders bevorzugt weniger als 5,4,3,2 oder 1 auf.

Unter "Prodrugs" von Rotigotin werden in dieser Patentanmeldung insbesondere Verbindungen verstanden, die im menschlichen Körper, insbesondere im Plasma oder beim Durchtritt durch Haut oder Schleimhaut in therapeutisch effektiver Menge zu Rotigotin gespalten, umgesetzt oder metabolisiert werden.

Rotigotin hat die Formel

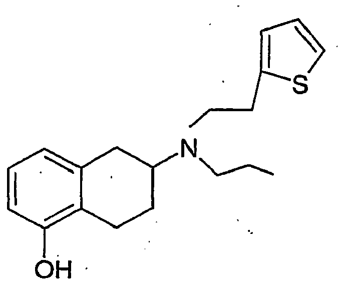

Prodrug sind daher Derivate der phenolischen Hydroxygruppe. Diese sind ausgewählt aus der Gruppe der Ester, z.b. Arylcarbonylester, Alkylcarbonylester oder Cycloalkylcarbonylester, insbesondere Alkylcarbonylester und Cycloalkylcarbonylester mit bis zu 6 Kohlenstoffatomen; Carbamate; Acetale; Ketale; Phosphate; Phosphonate; Sulfate; Sulfonate; und Silylether.

Der Begriff "Alkylcarbonylester" umfasst Verbindungen, in denen jeweils das Sauerstoffatom des Rotigotins an die Gruppe -C(O)-Alkyl gebunden ist.

Der Begriff "Cycloalkylcarbonylester" umfasst Verbindungen, in denen jeweils das Sauerstoffatom des Rotigotins an die Gruppe -C(O)-Cycloalkyl gebunden ist.

Der Begriff "Arylcarbonylester" umfasst Verbindungen, in denen jeweils das Sauerstoffatom des Rotigotins an die Gruppe -C(O)-Aryl gebunden ist.

Der Begriff "Carbamat" umfasst Verbindungen, in denen jeweils das Sauerstoffatom des Rotigotins an die Gruppe-C(O)-NRR1, -C(O)-NH-R1 oder-C(O)-NH₂ gebunden ist.

Der Begriff "Acetal" umfasst Verbindungen, in denen jeweils das Sauerstoffatom des Rotigotins an die Gruppe -CH(OR)R1 gebunden ist.

Der Begriff "Ketal" umfasst Verbindungen, in denen jeweils das Sauerstoffatom des Rotigotins an die Gruppe -C(OR)R1 R2 gebunden ist.

Der Begriff "Phosphat" umfasst Verbindungen, in denen jeweils das Sauerstoffatom des Rotigotins an die Gruppe -P(O₂H)OR gebunden ist.

Der Begriff "Phosphonat" umfasst Verbindungen, in denen jeweils das Sauerstoffatom des Rotigotins an die Gruppe -P(O₂H)R gebunden ist.

Der Begriff "Sulfat" umfasst Verbindungen, in denen jeweils das Sauerstoffatom des Rotigotins an die Gruppe -S(O)₂OR gebunden ist.

Der Begriff "Sulfonat" umfasst Verbindungen, in denen jeweils das Sauerstoffatom des Rotigotins an die Gruppe -S(O)₂R gebunden ist.

In den obigen Definitionen der Prodrugs sind R, R1 R2 jeweils unabhängig ausgewählt aus Wasserstoff, Alkyl, Cycloalkyl oder Aryl, und bevorzugt aus der Gruppe C1-6 Alkyl, C3-10 Cycloalkyl, und Phenyl.

R3 ist Alkyl, insbesondere C1-6 Alkyl.

"Alkyl" kann eine verzweigte oder unverzweigte Alkylgruppe sein, die vorzugsweise 1 bis 10 C-Atome, besonders bevorzugt 1 bis 6 C-Atome aufweist. Alkylgruppen können zusätzlich mit einem oder mehreren Substituenten substituiert sein, beispielsweise mit Halogen.

"Cycloalkyl" ist eine Alkylgruppe, die nur aus reinen ringbildenden C-Atomen bestehen kann oder optional weitere verzweigende C-Atome tragen kann. Bevorzugte Kettenlängen sind 3-10, besonders bevorzugt 4-8 oder 4-6 C-Atome.

"Aryl" ist bevorzugt Phenyl. Phenyl kann gegebenenfalls zusätzlich in einer oder mehreren Positionen substituiert sein, z.B. mit Alkoxy, Alkyl, Halogen oder Nitro.

Die Herstellung von Prodrugs durch Reaktion von Rotigotin mit entsprechenden reaktiven Vorstufen wie Säurechloriden, Säureanhydriden, Carbamylchloriden, Sulfonylchloriden etc. ist dem Fachmann auf dem Gebiet der klinischen Chemie bekannt und lässt sich der einschlägigen Fachliteratur entnehmen. Beispiele für Literaturstellen sind Bundgaard: Design of Prodrugs, Elsevier, Amsterdam, 1985; Higuchi und Stella: Pro-drugs as novel drug delivery systems in American Chemical Society, Washington DC, 1975; Sloan: Prodrugs - Topical and Ocular Drug Delivery, Ed: M. Dekker, 1992; Roche: Design of biopharmaceutical properties through prodrugs and analogs, Washington, DC, 1977.

Verschiedene Prodrugs des Razemats von Rotigotin (N-0437) sind beispielsweise beschrieben in Den Haas et al, Naunyn-Schmiedeberg's Arch Pharmacol 342, 1990, 655; Den Haas et al, Naunyn Schmiedebergs Arch Pharmacol. 341, 1990, 186 und Den Haas et al, J Pharm Pharmacol 43, 1991,11.

Die grundsätzliche Eignung eines Rotigotin-Derivats als Prodrug kann bestimmt werden, indem die jeweilige Verbindung unter definierten Bedingungen mit einem Enzymcocktail, einem Zellhomogenisat oder einer enzymhaltigen Zellfraktion inkubiert wird und das entstehende Rotigotin gemessen wird. Eine geeignete Enzymmischung ist beispielsweise enthalten in der S 9-Leberpräparation der Firma Gentest, Woburn, Ma, USA.

Alternativ kann eine inkubation mit Frischblut oder Plasma oder aber eines Homogenates der Unterhaut erfolgen, um eine leberunabhängige Metabolisierung der Prodrugs zur Wirkkomponente zu demonstrieren. Für transdermale Applikation ist eine in vitro Untersuchung der Permeation an exzidierter Haut notwendig. Der endgültige Nachweis der Eignung und potentiellen Wirksamkeit in den Krankheitsmodellen erfolgt durch eine Bestimmung des aus dem Prodrug gebildeten 2-N-Propylamino-5-hydroxytetralins im Plasma.

In-vivo sollte ein Prodrug soviel Rotigotin freisetzen, dass eine therapeutisch/prophylaktisch effektive steady-state Konzentration Rotigotin im Plasma erreicht wird. Als effektive Konzentrationen werden dabei im allgemeinen Rotigotinkonzentrationen zwischen 0.01 und 50 ng/mL, bevorzugt zwischen 0.05 ng und 20 ng/mL und besonders bevorzugt zwischen 0.1 und 10 ng/mL Plasma angesehen.

Rotigotin ist das S(-)-Enantiomer von 5,6,7,8-Tetrahydro-6-[propyl[2-(2-thienyl)ethyl] amino]-1-naphthol. Dies bedeutet, dass der Anteil des in Parkinsonmodellen inaktiven (R)-Enantiomers im Arzneimittel erfindungsgemäß gering ist. Das (R)-Enantiomer liegt bevorzugt mit einem Anteil von < 10 Mol%, besonders bevorzugt mit einem Anteil von < 2 Mol% und ganz besonders bevorzugt mit einem Molanteil von < 1 %, bezogen auf die Gesamtmenge Rotigotin, im Arzneimittel vor.

Rotigotin und seine Prodrugs können als freie Basen oder in Form der physiologisch akzeptablen Salze, z.B. in Form des Hydrochlorids, im Arzneimittel vorliegen.

"Physiologisch akzeptable Salze" schließen nicht-toxische Additionssalze des Rotigotins mit organischen oder anorganischen Säuren ein. Ein bevorzugtes Beispiel eines geeigneten Salzes ist das HCl Salz.

Zur Verabreichung von Rotigotin und seinen Prodrugs stehen verschiedene Applikationswege zur Verfügung, die der Fachmann je nach Bedarf, Zustand und Alter des Patienten, erforderlicher Dosierung und gewünschtem Applikationsintervall auswählen und anpassen kann.

Eine bevorzugte Art der Verabreichung von Rotigotin ist die transdermale Gabe. Die Darreichungsform kann grundsätzlich ausgewählt sein aus z.B. Salbe, Paste, Spray, Folie, Pflaster oder einer iontophoretischen Vorrichtung.

Bevorzugt wird Rotigotin dabei in Pflasterform auf die Haut des Patienten gebracht, wobei der Wirkstoff bevorzugt in einer Matrix aus adhesivem Polymer, z.B. einem selbstklebendem adhesivem Polysiloxan, vorliegt. Beispiele für geeignete transdermale Formulierungen finden sich in WO 99/49852, WO 02/89777, WO 02/89778, WO 2004/058247 und WO 2004/012721 sowie in Ausführungsbeispiel 1. Eine solche Darreichungsform ermöglicht die Einstellung eines weitgehend konstanten Plasmaspiegels und damit eine konstante dopaminerge Stimulation über das gesamte Applikationsintervall (WO 02/89778; Metman, Clinical Neuropharmacol. 24, 2001, 163).

Wird dagegen ein Arzneimittel in Form einer subkutanen oder intramuskularen Depotform gewünscht, kann das Rotigotin beispielsweise als Salzkristall, z.B. als kristallines Hydrochlorid, in einem hydrophobem, wasserfreiem Medium suspendiert und injiziert werden, wie in WO 02/15903 und Ausführungsbeispiel 2 beschrieben.

Rotigotin kann grundsätzlich auch in Form von Mikrokapseln, Mikropartikeln oder Implantaten auf Basis bioabbaubarer Polymere, wie beispielsweise in WO 02/38646 beschrieben, verabreicht werden.

Andere denkbare Formen der Verabreichung von Rotigotin und seinen Prodrugs sind transmukosale Formulierungen, z.B. Sublingualsprays, rektale Formulierungen oder Aerosole zur pulmonalen Verabreichung.

Geeignete Dosierungen von Rotigotin liegen zwischen 0,05 und ca. 50 mg/Tag, wobei vorzugsweise Tagesdosen zwischen 0,1 und 40 mg und insbesondere zwischen 0,2 und 20 mg/Tag verabreicht werden. Dabei kann die Dosierung einschleichend erfolgen, das heißt, die Behandlung kann gegebenenfalls mit niedrigen Dosierungen beginnen, die dann bis zur Erhaltungsdosis gesteigert werden.

Dem Fachmann ist klar, dass das Dosierungsintervall in Abhängigkeit von der applizierten Menge, der Applikationsart und dem Tagesbedarf des Patienten variieren kann. So kann eine transdermale Applikationsform beispielsweise zur einmal täglichen, dreitägigen oder siebentägigen Verabreichung konzipiert sein, während ein subkutanes oder intramuskuläres Depot Injektionen beispielsweise im Ein-, Zwei- oder Vierwochen-Rhythmus ermöglichen kann.

In der neuroprotektiven Arzneiform können neben Rotigotin noch andere Wirkstoffe vorliegen, die die Progression des dopaminergen Zellverlusts verhindern.

Beispiele hierfür sind antiapoptotisch wirksame Substanzen (Minocycline, FK-506, Cyclosporin A, zVAD) sowie Neurotrophine, wie z.B. der Glial-cell-derived neurotrophic factor (GDNF).

In einem Kombinationspräparat kann eine sequentielle Gabe beispielsweise erreicht werden, indem eine Darreichungsform, z.B. eine orale Tablette, zwei unterschiedliche Schichten mit differierendem Freisetzungsprofil für die verschiedenen pharmazeutisch aktiven Bestandteile aufweist. Dem Fachmann ist klar, dass im Kontext der vorliegenden Erfindung verschiedene Darreichungsformen und Applikationsschemata denkbar sind, die alle Gegenstand der Erfindung sind.

### Ausführungsbeispiele:

### Ausführungsbeispiel 1: Rotiaotin-Pflaster

1.8 g Rotigotin (freie Base) werden in 2.4 g Ethanol gelöst und zu 0.4 g Kollidon 90F (gelöst in 1g Ethanol) gegeben. Diese Mischung wird zu einer 74%igen Lösung von Silikonpolymeren (8.9 g BioPSA 7-4201 + 8.9 g BIO-PSA 7-4301 [Dow Corning]) in Heptan gegeben. Nach Zugabe von 2.65 g Petrolether wird die Mischung für 1 Stunde bei 700 UpM gerührt um eine homogene Dispersion zu erhalten. Nach Laminierung auf Polyester wurde bei 50°C getrocknet. Das Pflastergewicht betrug schließlich 50 g/cm2.

### Ausführungsbeispiel 2: Rotigotin-Depotsuspensionen

(a) 1411,2 g Miglyol 812 wurde in eine Duran Flasche eingewogen. 14,4 g Imwitor 312 wurde dem Miglyol zugegeben und im Anschluß für 30 Minuten unter Rühren auf 80°C erwärmt. Die klare Lösung wurde auf Raumtemperatur abgekühlt und gefiltert.
(b) 1188 g der unter (a) hergestellten Lösung wurde in einen Glaslaborreaktor überführt, 12 g Rotigotin zugesetzt und für 10 Minuten mit einem Ultraturrax bei 10.000 UpM unter Stickstoff homogenisiert. Die Suspension wurde bei laufendem Ultraturrax (2.000 UpM) in Braunglasflaschen abgefüllt.

### Ausführungsbeispiel 3: Subakutes MPTP-Modell

Zur Intoxikation werden Mäusen 80 mg/kg des Neurotoxins 1-Methyl-4-phenyl-1,2,3,6-tetrahydro-pyridine (MPTP) verabreicht (in Portionen von 20 mg/kg in zweistündigen Abständen, Gruppe 3-6 in Abbildung 1 und 2), was dazu führt, dass ca. 50 - 60 % der Neurone der Substantia nigra degenerieren (maximale Degeneration in Gruppe 3 in Abbildung 1 und 2). Rotigotin wird täglich in Dosen zu je 0.3, 1 oder 3 mg/kg über 7 Tage als sogenannte 'Slow-Release-Formulierung' (siehe Ausführungsbeispiel 2) verabreicht (Gruppe 4-6 in Abbildung 1 und 2). Eine Gruppe MPTP-behandelter Tiere (Gruppe 3) erhält Rotigotin-Vehikel-Lösung (siehe Ausführungsbeispiel 2 ohne Rotigotin HCl) und dient als Referenz. Als Kontrollen dienen die Gruppen 1, 2 und 7, wobei Gruppe 1 keinerlei Behandlung erfährt, Gruppe 2 mit den Vehikel-Lösungen für MPTP und Rotigotin behandelt wird und Gruppe 7 ausschließlich Rotigotin erhält. Am 8. Tag werden die Tiere getötet, die Gehirne entnommen und eingefroren. Gefrierschnitte werden mit 100 pm [¹²⁵I] PE21 ([¹²⁵I]-(E)-N(3-iodoprop-2-enyl)-2β-carboxymethyl-3β-(4'-methylphenyl)-nortropane) in Phosphat Puffer, pH 7.4, inkubiert, um die Menge der im Striatum noch vorhandenen Dopamin-Transporter zu markieren, was als Indiz für die Menge funktionierender Nervenendigungen dient. Rotigotin verbessert das Überleben der Neurone und ihrer Nervenendigungen dosisabhängig. Dies ist ein klarer Hinweise für die neuroprotektiven Eigenschaften der Substanz (Abbildung 1 und 2).

### Ausführungsbeispiel 4: Akutes MPTP-Modell (einschließlich Apoptose)

Zur Intoxikation werden Mäusen 80 mg/kg des Neurotoxins 1-Methyl-4-phenyl-1,2,3,6-tetrahydro-pyridine (MPTP) verabreicht (in Portionen von 20 mg/kg in zweistündigen Abständen), was dazu führt, dass ca. 50 - 60 % der Neurone der Substantia nigra degenerieren. Ca 16. Stunden vorher wird Rotigotin in Dosen zu je 0.3, 1 oder 3 mg/kg als sogenannte 'Slow-Release-Formulierung' verabreicht. Diffusions- und Absorptionslatenzen führen dazu, daß Rotigotin dann optimal verfügbar ist, wenn MPTP gegeben wird. Nach 24 Stunden werden die Tiere getötet und die Gehirne fixiert. Die Gehirnschnitte werden mit FluoroJade zur Identifzierung degenerierender Zellen gefärbt. Die immunhistochemische Markierung der Tyrosin-Hydroxylase dient der Identifizierung dopaminerger Neurone. Die Färbung der Tyrosin-Hydroxylase ergibt keine Unterschiede zwischen behandelten und unbehandelten Tieren; die Färbung mit FluoroJade zeigt eine große Zahl degenerierender Neurone; die Neurone sind allerdings noch nicht vollständig entfernt; dies legt nahe, dass der Zelluntergang apoptotisch verläuft. Die Anzahl der degenerierenden Neurone ist um ca. 50 % geringer nach Applikation von Rotigotin, was die neuroprotektive Eigenschaft der Substanz weiter belegt (Tabelle 1).

### Ausführungsbeispiel 5: Bestimmung des motorischen UPDRS-Scores

Der motorische UPDRS Score (Teil III des UPDRS-Scores) wird durch Untersuchung der Patienten anhand der in der folgenden Tabelle 2 wiedergegebenen Kriterien 18-31 bestimmt, wobei die sich aus den jeweiligen Kriterien ergebenden Punktwerte jeweils aufaddiert werden.

### III. MOTORISCHE UNTERSUCHUNG

### 18. Sprache:

| | | |
|---|---|---|
| □ | 0 - | Normal. |
| □ | 1 - | Leichte Abnahme von Ausdruck, Diktion und/oder Volumen. |
| □ | 2 - | Monoton, verwaschen, aber verständlich; mäßig behindert. |
| □ | 3 - | Deutliche Beeinträchtigung, schwer zu verstehen. |
| □ | 4 - | Unverständlich. |

### 19. Gesichtsausdruck:

| | | |
|---|---|---|
| □ | 0 - | Normal. |
| □ | 1 - | Minimal veränderte Mimik, könnte ein normales " Pokergesicht" sein. |
| □ | 2 - | Leichte, aber eindeutig abnorme Verminderung des Gesichtsausdruckes. |
| □ | 3 - | Mäßig verminderte Mimik; Lippen zeitweise geöffnet. |
| □ | 4 - | Maskenhaftes oder erstarrtes Gesicht mit stark oder völlig fehlendem Ausdruck; Lippen stehen um 7 mm auseinander. |

### 20. Ruhetremor: (G = Gesicht, RH = rechte Hand, LH = linke Hand, RF = rechter Fuß, LF = linker Fuß)

| G | RH | LH | RF | LF | | |
|---|---|---|---|---|---|---|
| □ | □ | □ | □ | □ | 0 - | Keine. |
| □ | □ | □ | □ | □ | 1 - | Leicht und selten vorhanden. |
| □ | □ | □ | □ | □ | 2 - | Geringe Amplitude persistierend; oder mäßige Amplitude, aber nur intermittierend auftretend. |
| □ | □ | □ | □ | □ | 3 - | Mäßige Amplitude, die meiste Zeit vorhanden. |
| □ | □ | □ | □ | □ | 4 - | Ausgeprägte Amplitude, die meiste Zeit vorhanden. |

### 21. Aktions- oder Haltungstremor der Hände: (R = rechts, L = links)

| R | L | | |
|---|---|---|---|
| □ | □ | 0 - | Fehlt. |
| □ | □ | 1 - | Leicht; bei Bewegung vorhanden. |
| □ | □ | 2 - | Mäßige Amplitude, bei Bewegung vorhanden. |
| □ | □ | 3 - | Mäßige Amplitude, bei Beibehalten der Haltung und bei Bewegung vorhanden. |
| □ | □ | 4 - | Ausgeprägte Amplitude; beim Essen störend. |

### 22. Rigidität: (Geprüft bei passiver Bewegung der großen Gelenke am sitzenden Patienten. Zahnradphänomen kann ignoriert werden). (N = Nacken, ROE = rechte obere Extremität, LOE = linke obere Extremität, RUE = rechte untere Extremität, LUE = linke untere Extremität)

| N | ROE | LOE | RUE | LUE | | |
|---|---|---|---|---|---|---|
| □ | □ | □ | □ | □ | 0 - | Fehlt. |
| □ | □ | □ | □ | □ | 1 - | Leicht oder nur erkennbar bei Aktivierung durch spiegelbildliche oder andere Bewegungen. |
| □ | □ | □ | □ | □ | 2 - | Leicht bis mäßig. |
| □ | □ | □ | □ | □ | 3 - | Ausgeprägt, jedoch voller Bewegungsumfang bleibt erreicht. |
| □ | □ | □ | □ | □ | 4 - | Stark; Schwierigkeit beim Ausführen aller Bewegungen. |

### 23. Fingerklopfen: (Patient berührt in rascher Reihenfolge und bei größtmöglicher Amplitude und mit jeder Hand gesondert den Daumen mit dem Zeigefinger). (R= rechts, L = links).

| R | L | | |
|---|---|---|---|
| □ | □ | 0 - | Normal. |
| □ | □ | 1 - | Leichte Verlangsamung und/oder-Verringerung der Amplitude. |
| □ | □ | 2 - | Mäßig eingeschränkt. Eindeutige und frühzeitige Ermüdung. Bewegung kann gelegentlich unterbrochen werden. |
| □ | □ | 3 - | Stark eingeschränkt. Verzögerter Start der Bewegungen oder Unterbrechung fortlaufender Bewegungen. |
| □ | □ | 4 - | Kann die Aufgabe kaum ausführen. |

### 24. Handbewegungen: (Patient öffnet und schließt die Hände in rascher Reihenfolge bei größtmöglicher Amplitude und mit jeder Hand gesondert). (R = rechts, L = links)

| R | L | | |
|---|---|---|---|
| □ | □ | 0 - | Normal. |
| □ | □ | 1 - | Leichte Verlangsamung und/oder Verringerung der Amplitude. |
| □ | □ | 2 - | Mäßig eingeschränkt. Eindeutige und frühzeitige Ermüdung. Bewegung kann gelegentlich unterbrochen werden. |
| □ | □ | 3 - | Stark eingeschränkt. Verzögerter Start der Bewegungen oder Unterbrechung fortlaufender Bewegungen. |
| □ | □ | 4 - | Kann die Aufgabe kaum ausführen. |

### 25. Rasch wechselnde Bewegungen der Hände: (Pronation-Supinationsbewegungen der Hände, vertikal oder horizontal, mit größtmöglicher Amplitude, beide Hände gleichzeitig).

| R | L | | |
|---|---|---|---|
| □ | □ | 0 - | Normal. |
| □ | □ | 1 - | Leichte Verlangsamung und/oder Verringerung der Amplitude. |
| □ | □ | 2 - | Mäßig eingeschränkt. Eindeutige und frühzeitige Ermüdung. Bewegung kann gelegentlich unterbrochen werden. |
| □ | □ | 3 - | Stark eingeschränkt. Verzögerter Start der Bewegungen oder Unterbrechung fortlaufender Bewegungen. |
| □ | □ | 4 - | Kann die Aufgabe kaum ausführen. |

### 26. Agilität der Beine: (Der Patient klopft in rascher Reihenfolge mit der Ferse auf den Boden und hebt dabei das ganze Bein an. Die Amplitude soll mindestens 7,5 cm betragen.

| R | L | | |
|---|---|---|---|
| □ | □ | 0 - | Normal. |
| □ | □ | 1 - | Leichte Verlangsamung und/oder Verringerung der Amplitude. |
| □ | □ | 2 - | Mäßig eingeschränkt. Eindeutige und frühzeitige Ermüdung. Bewegung kann gelegentlich unterbrochen werden. |
| □ | □ | 3 - | Stark eingeschränkt. Verzögerter Start der Bewegungen oder Unterbrechung fortlaufender Bewegungen. |
| □ | □ | 4 - | Kann die Aufgabe kaum ausführen. |

### 27. Aufstehen vom Stuhl: (Patient versucht mit vor der Brust verschränkten Armen von einem geradelehnigen Holz- oder Metallstuhl aufzustehen).

| | | |
|---|---|---|
| □ | 0 - | Normal. |
| □ | 1 - | Langsam; kann mehr als einen Versuch benötigen. |
| □ | 2 - | Stößt sich an den Armlehnen hoch. |
| □ | 3 - | Neigt zum Zurückfallen und muß es eventuell mehrmals versuchen, kann jedoch ohne Hilfe aufstehen. |
| □ | 4 - | Kann ohne Hilfe nicht aufstehen. |

### 28. Haltung:

| | | |
|---|---|---|
| □ | 0 - | Normal aufrecht. |
| □ | 1 - | Nicht ganz aufrecht, leicht vorgebeugte Haltung; könnte bei einem älteren Menschen normal sein. |
| □ | 2 - | Mäßig vorgebeugte Haltung; eindeutig abnorm, kann leicht zu einer Seite geneigt sein. |
| □ | 3 - | Stark vorgebeugte Haltung mit Kyphose; kann mäßig zu einer Seite geneigt sein. |
| □ | 4 - | Ausgeprägte Beugung mit extrem abnormer Haltung. |

### 29. Gang:

| | | |
|---|---|---|
| □ | 0 - | Normal. |
| □ | 1 - | Geht langsam, kann einige kurze Schritte schlurfen, jedoch keine Festination oder Propulsion. |
| □ | 2 - | Gehen schwierig, benötigt aber wenig oder keine Hilfe; eventuell leichtes Trippeln, kurze Schritte oder Propulsion. |
| □ | 3 - | Starke Gehstörung, benötigt Hilfe. |
| □ | 4 - | Kann überhaupt nicht gehen, auch nicht mit Hilfe. |

### 30. Haltungsstabilität: (Reaktion auf plötzliches Verlagern nach hinten durch Ziehen an den Schultern des Patienten; der mit geöffneten Augen und leicht auseinanderstehenden Füßen geradesteht. Der Patient ist darauf vorbereitet).

| | | |
|---|---|---|
| □ | 0 - | Normal. |
| □ | 1 - | Retropulsion, gleicht aber ohne Hilfe aus. |
| □ | 2 - | Fehlen einer Haltungsreaktion; würde fallen, wenn er nicht vom Untersucher aufgefangen würde. |
| □ | 3 - | Sehr instabil; neigt dazu, spontan das Gleichgewicht zu verlieren. |
| □ | 4 - | Kann nicht ohne Unterstützung stehen. |

### 31. Bradykinesie und Hypokinesie des Körpers: (Kombination aus Langsamkeit, Zögern, verminderten Mitbewegungen der Arme, geringe Bewegungsamplitude und allgemeine Bewegungsarmut)

| | | |
|---|---|---|
| □ | 0 - | Keine. |
| □ | 1 - | Minimale Verlangsamung, Bewegung wirkt beabsichtigt; könnte bei manchen Menschen normal sein. Möglicherweise herabgesetzte Amplitude. |
| □ | 2 - | Leichte Verlangsamung und Bewegungsarmut, die eindeutig abnorm sind. Alternativ auch herabgesetzte Amplitude. |
| □ | 3 - | Mäßige Verlangsamung und Bewegungsarmut oder Herabsetzung der Amplitude. |
| □ | 4 - | Ausgeprägte Verlangsamung, Bewegungsarmut oder Herabsetzung der Amplitude. |

### Ausführungsbeispiel 6: In vitro Umsetzung eines Prodrugs in die Wirksubstanz

Aus Leberzellhomogenaten von Mensch, Affe, Hund, Ratte oder Maus wird durch differentielle Zentrifugation die Mikrosomenfraktion erhalten, die die wesentlichen metabolisierenden Enzyme enthält; alternativ kann auch die zytoplasmatische Fraktion gewonnen werden. Die subzelluläre Fraktion wird mit einem Puffer so suspendiert, dass eine Lösung mit einem definierten Proteingehalt erhalten wird. Nach Zufügen von 1 µM des zu testenden Prodrugs erfolgt die Inkubation bei 37 °C für 60 min. Anschließend wird Rotigotin mittels HPLC/UV oder auch mittels HPLC/MS quantifiziert und zur eingesetzten Menge in Beziehung gesetzt. Für detailliertere Analysen werden Konzentrations- oder Zeitreihen untersucht.

## Patentansprüche

1. Rotigotin, seine Salze oder Prodrugs davon zur Verwendung bei der Behandlung von Erkrankungen, die mit einem erhöhten dopaminergen Zelluntergang verbunden sind,
wobei die Behandlung an Individuen erfolgt, die ausgewählt sind aus der Gruppe
(a) von Individuen ohne Morbus Parkinson-Symptome, aber mit einem erhöhten Risiko für Morbus Parkinson, wobei die Individuen eine Mutation in einem PARK-Gen aufweisen und/oder
(b) von Individuen bei denen mindestens drei der vier Kardinalsymptome von Morbus Parkinson (Rigor, Ruhetremor, Bradykinesie, posturale Instabilität) noch nicht vorhanden sind,
wobei das Prodrug ausgewählt ist aus der Gruppe der Ester, Carbamate, Acetale, Ketale, Phosphate, Phosphonate, Sulfate, Sulfonate und Silylether.

2. Rotigotin, seine Salze oder Prodrugs davon zur Verwendung nach Anspruch 1, worin die Erkrankung, die mit einem erhöhten dopaminergen Zelluntergang verbunden ist, ausgewählt ist aus alpha-Synucleopathien, REM-Schlafstörungen und Störungen des Geruchssinns.

3. Rotigotin, seine Salze oder Prodrugs davon zur Verwendung nach einem der vorhergehenden Ansprüche, wobei den Individuen ein oder mehrere der folgenden klinischen Frühsymptome zugeordnet werden können: Riechstörungen, Depressionen, Schlafstörungen vom Typ der 'REM Sleep Behaviour Disorders', Obstipationen und kurzfristige Bewegungsanomalien.

4. Rotigotin, seine Salze oder Prodrugs davon zur Verwendung nach einem der vorhergehenden Ansprüche, wobei die Individuen eine Mutation in einem PARK-Gen und/oder Veränderungen des alpha-Synuclein oder Neuromelanin-Musters aufweisen.

5. Rotigotin, seine Salze oder Prodrugs davon zur Verwendung nach einem der vorangehenden Ansprüche, wobei die Individuen vor Beginn der Arzneimittelgabe einen Verlust dopaminerger Zellen in der Substantia nigra von weniger als 60% aufweisen.

6. Rotigotin, seine Salze oder Prodrugs davon zur Verwendung nach einem der vorangehenden Ansprüche, wobei die Individuen vor Beginn der Arzneimittelgabe einen motorischen UPDRS-Score von weniger als 9 aufweisen.

7. Rotigotin, seine Salze oder Prodrugs davon zur Verwendung nach einem der vorangehenden Ansprüche, wobei die Individuen einen Höhn-Yahr-Score von 0 aufweisen.

8. Rotigotin, seine Salze oder Prodrugs davon zur Verwendung nach einem der vorhergehenden Ansprüche, wobei das Arzneimittel zur parenteralen, transdermalen oder mukosalen Administration vorgesehen ist.

9. Rotigotin, seine Salze oder Prodrugs davon zur Verwendung nach einem der vorhergehenden Ansprüche, wobei das Rotigotin in einer Dosierung von 0,05 bis 50 mg pro Tag verabreicht wird.

10. Rotigotin, seine Salze oder Prodrugs davon zur Verwendung nach einem der vorhergehenden Ansprüche, wobei das Arzneimittel neben Rotigotin außerdem eine antiapoptotisch wirksame Substanz, wie Minocycline, FK-506, Cyclosporin A oder zVAD, und/oder Neurotrophine umfasst.

## Claims

1. Rotigotine, its salts or prodrugs thereof for use in the treatment of diseases which are associated with increased dopaminergic cellular decay, wherein the treatment is effected on individuals who are selected from the group
(a) of individuals without symptoms of Parkinson's disease, but having an increased risk of Parkinson's disease, wherein the individuals have a mutation in a PARK gene and/or
(b) of individuals in which at least three of the four cardinal symptoms of Parkinson's disease (rigidity, passive tremor, bradykinesia, postural instability) are not yet present,
wherein the prodrug is selected from the group of esters, carbamates, acetals, petals, phosphates, phosphonates, sulphates, sulphonates and silyl ethers.

2. Rotigotine, its salts or prodrugs thereof for use according to claim 1, wherein the disease which is associated with increased dopaminergic cellular decay is selected from alpha-synucleopathies, REM sleep disturbances and disturbances of the sense of smell.

3. Rotigotine, its salts or prodrugs thereof for use according to one of the preceding claims, wherein one or more of the following early clinical symptoms may be assigned to the individuals: olfactory disturbances, depression, sleep disturbances of the type REM Sleep Behaviour Disorders', constipation and short-term motor anomalies.

4. Rotigotine, its salts or prodrugs thereof for use according to one of the preceding claims, wherein the individuals have a mutation in a PARK gene and/or changes in the alpha-synuclein or neuromelanine pattern.

5. Rotigotine, its salts or prodrugs thereof for use according to one of the preceding claims, wherein before the start of medicament administration the individuals have a loss of dopaminergic cells in the substantia nigra of less than 60%.

6. Rotigotine, its salts or prodrugs thereof for use according to one of the preceding claims, wherein before the start of medicament admmistration the individuals have a motor UPDRS score of less than 9.

7. Rotigotine, its salts or prodrugs thereof for use according to one of the preceding claims, wherein the individuals have a Höhn-Yahr score of 0.

8. Rotigotine, its salts or prodrugs thereof for use according to one of the preceding claims, wherein the medicament is intended for parenteral, transdermal or mucosal administration.

9. Rotigotine, its salts or prodrugs thereof for use according to one of the preceding claims, wherein the rotigotine is administered in a dose of 0.05 to 50 mg per day.

10. Rotigotine, its salts or prodrugs thereof for use according to one of the preceding claims, wherein the medicament comprises, in addition to rotigotine, also an anti-apoptotically effective substance, such as minocycline, FK-506, cyclosporin A or zVAD, and/or neurotrophines.

## Revendications

1. Rotigotin, ses sels ou ses promédicaments à utiliser dans le traitement de maladies associées à une augmentation de la mort des cellules dopaminersiques,
le traitement étant effectué sur des sujets choisis dans le groupe
(a) des sujets sans symptômes de la maladie de Parkinson mais à risque accru de maladie de Parkinson, lesquels sujets présentent une mutation d'un gène PARK, et/ou
(b) des sujets qui ne présentent pas encore au moins trois des quatre symptômes cardinaux de la maladie de Parkinson (rigidité, tremblements au repos, bradykinésie, instabilité posturale),
le promédicament étant choisi parmi les esters, carbamates, acétals, cétals, phosphates, phosphonates, sulfates, sulfonates et éthers sylyliques.

2. Rotigotine, ses sels ou ses promédicaments pour une utilisation selon la revendication 1, dans laquelle la maladie associée à une augmentation de la mort des cellules dopaminergiques est choisie parmi les synucléopathies alpha, les troubles du sommeil paradoxal et les troubles de l'odorat.

3. Rotigotine, ses sels ou ses promédicaments pour une utilisation selon l'une quelconque des revendications précédentes, dans laquelle un ou plusieurs des symptômes cliniques précoces suivants peuvent être attribués aux sujets : troubles de l'odorat, dépression, troubles du sommeil du type des "troubles du comportement en sommeil paradoxal", constipation et anomalies motrices de courte durée.

4. Rotigotine, ses sels ou ses promédicaments pour une utilisation selon l'une quelconque des revendications précédentes, dans laquelle les sujets présentent une mutation d'un gène PARK et/ou des altérations du statut de synucléine alpha ou de neuromélanine.

5. Rotigotine, ses sels ou ses promédicaments pour une utilisation selon l'une quelconque des revendications précédentes, dans laquelle les sujets présentent, avant le début de l'administration du médicament, une perte de cellules dopaminergiques dans la substance noire de moins de 60 %.

6. Rotigotine, ses sels ou ses promédicaments pour une utilisation selon l'une quelconque des revendications précédentes, dans laquelle les sujets présentent, avant le début de l'administration du médicament, un score moteur UPDRS inférieur à 9.

7. Rotiaotine, ses sels ou ses promédicaments pour une utilisation selon l'une quelconque des revendications précédentes, dans laquelle les individus présentent un score de Hoehn et Yahr de 0.

8. Rotigotine, ses sels ou ses promédicaments pour une utilisation selon l'une quelconque des revendications précédentes, dans laquelle le médicament est destiné à une administration parentérale, transdermique ou muqueuse.

9. Rotigotine, ses sels ou ses promédicaments pour une utilisation selon l'une quelconque des revendications précédentes, dans laquelle la rotigotine est administrée selon un dosage de 0,05 à 50 mg par jour.

10. Rotigotine, ses sels ou ses promédicaments pour une utilisation selon l'une quelconque des revendications précédentes, dans laquelle le médicament contient, outre la rotigotine, une substance ayant une action antiapoptotique, telle que la minocycline, le FK-506, la ciclosporine A ou le zVAD, et/ou les neurotrophines.
